Europäisches Patentamt

European Patent Office

Office européen des brevets

(11) Publication number: **0 487 404 B1**

# EUROPEAN PATENT SPECIFICATION

(45) Date of publication of patent specification: **11.10.95**    (51) Int. Cl.⁶: **A61K 7/48**, A61K 31/70

(21) Application number: **91403107.5**

(22) Date of filing: **19.11.91**

(54) **External dermatological composition.**

(30) Priority: **19.11.90 JP 311319/90**
           **19.11.90 JP 311320/90**

(43) Date of publication of application:
**27.05.92 Bulletin 92/22**

(45) Publication of the grant of the patent:
**11.10.95 Bulletin 95/41**

(84) Designated Contracting States:
**DE FR GB IT**

(56) References cited:
**EP-A- 0 398 484**
**EP-A- 0 425 066**

**PATENT ABSTRACTS OF JAPAN vol. 8, no. 114 (C-225)(1551) 26 May 1984 & JP- A-59 027 825 ( SUNSTAR HAMIGAKI K.K. ) 14 February 1984**

**PATENT ABSTRACTS OF JAPAN vol. 13, no. 472 (C-647)(3820) 25 October 1989 & JP-A-1 186 811 (Sunstar Inc.) 26 July 1989**

(73) Proprietor: **KABUSHIKI KAISHA HAYASHIBARA SEIBUTSU KAGAKU KEN-KYUJO**
**2-3, 1-chome, Shimoishii**
Okayama-shi
Okayama (JP)

(72) Inventor: **Sakamoto, Tetsuo**
**67-11-1, Suwa 1-chome**
**Tama-shi,**
**Tokyo (JP)**
Inventor: **Tamaki, Shuya**
**129, Mamedo-cho,**
**Kohoku-ku**
**Yokohama-shi,**
**Kanagawa (JP)**
Inventor: **Akiyama, Junnichi**
**8-18, Hiyodoridai 4-chome,**
**Kitaku**
**Kobe-shi,**
**Hyogo (JP)**
Inventor: **Miyake, Toshio**
**3-23, Ishima-cho 1-chome**
**Okayama-shi,**
**Okayama (JP)**

(74) Representative: **Rinuy, Santarelli**
**14, avenue de la Grande Armée**
**F-75017 Paris (FR)**

**Description**

BACKGROUND OF THE INVENTION

(1) Field of the Invention

The present invention relates to an external dermatological composition (or external skin treatment composition) having an excellent stability, which contains a vitamin C derivative exerting an excellent vitamin C activity in the living body. More particularly, the present invention relates to an external dermatological composition having a high safety and providing a highly improved effect of beautifying and whitening the skin and of preventing skin roughening.

(2) Description of the Related Art

Vitamin C has various medicinal effects such as a nourishing effect, an oxidation-preventing effect and an effect of cleaning and whitening the skin, and vitamin C is incorporated in medicines and endermic liniments, including non-pharmacopeial agents, but as vitamin C is very unstable, it is generally incorporated in the form of a vitamin C derivative.

As the derivative, there are currently used a fatty acid ester of L-ascorbic acid, L-ascorbic acid 2-sulfate and the like, but these derivatives are unstable and derivative-incorporated products become yellow to reddish brown. Moreover, even if the derivatives are stable, no vitamin C activity is manifested in the living body.

A skillful joint operation by water, a dermal lipid, an intercellular lipid and a water-soluble NMF component on the skin and within the skin is necessary for maintaining a water content of 10 to 20% in the keratin and exerting a normal biological function of the keratin, and it is said that if any one of these factors is deficient, skin roughening occurs. Accordingly, in external dermatological compositions, especially a base cosmetic, a moisture-retaining agent or a water-soluble NMF component is incorporated for supplementing these factors. Simultaneously, a skin roughening improver is generally incorporated for moderating once-occurring skin roughening.

As the skin roughening improver, there have been used amino acids or derivatives thereof, allantoin or derivatives thereof, dead nettle extract, glycyrrhizin, vitamin E or derivatives thereof, and mucopolysaccharides, but the skin roughening-moderating effect of these improvers is low, especially at low concentrations, and is not satisfactory. If the improvers are incorporated at high concentrations, the skin roughening-moderating effect is conspicuously enhanced, but stimula are given to the skin and a problem of safety arises.

SUMMARY OF THE INVENTION

Accordingly, the objects of the present invention are to eliminate the above-mentioned disadvantages of the prior art and to provide a vitamin C derivative stable for a long period and exerting a vitamin C activity in the living body.

Another object of the present invention is to provide an external dermatological composition having a synergistically improved skin-beautifying and whitening effect.

Still another object of the present invention it to provide an external dermatological composition comprising a convention skin roughening improver and having a synergistically enhanced skin roughening moderating effect.

Other objects and advantages of the present invention will be apparent from the following description.

In accordance with one aspect of the present invention, there is provided an external dermatological composition having whitening effects comprising (i) $\alpha$-glycosyl-L-ascorbic acid having no direct reducing property and (ii) at least one member selected from the group consisting of ethinylestradiol, kojiic acid, placental extract, amino acids and derivatives thereof, dead nettle extract, glycyrrhizin, and mucopolysaccharides, wherein respective contents of the components (i) and (ii) exluding ethinylestradiol, are 0.0005 to 10.0% by weight and 0.0005 to 20.0% by weight, and the content of ethinylestradiol is 0.00001 to 0.03% by weight.

DESCRIPTION OF THE PREFERRED EMBODIMENTS

The construction of the present invention will now be described.

The phrase "having no direct reducing property", as used in the present invention, means a property such that 2,6-dichlorophenolindophenol is not directly reduced and not decolored.

Of the α-Glycosyl-L-ascorbic acids having no direct reducing property, that can be used in the present invention, 2-O-α-D-glucosyl-L-ascorbic acid is most preferable.

The 2-O-α-glucosyl-L-ascorbic acid used in the present invention is described in Biochimica et Biophysica Acta, volume 1035, pages 44 through 50 (1990), and has a structure in which glucose is ether-bonded in the α-form to the OH-group at the 2-position of ascorbic acid.

The process for the preparation of the α-Glycosyl-L-ascorbic acid having no direct reducing property, as used in the present invention, is not particularly critical, and either a biochemical process or an organic chemical process can be adopted. From the viewpoints of safety, biological activity, and economical advantages, it is generally preferred that the α-Glycosyl-L-ascorbic acid is prepared by a biochemical process, for example, a process in which a solution containing L-ascorbic acid and a glycosyl saccharide compound is reacted with a saccharide transfer enzyme, as disclosed in Japanese Japanese Patent Application No. 01-27418 filed by the present applicant.

The α-Glycosyl-L-ascorbic acid thus prepared has the following characteristic properties.

(1) The α-glucosyl-L-ascorbic acid is very stable, and does not cause a Maillard reaction, unlike L-ascorbic acid. Accordingly, even if the α-Glycosyl-L-ascorbic acid is co-present with an amino acid, a peptide, a protein, a lipid, a saccharide or a physiologically active substance, the acid does not cause any useless reaction, but instead, stabilizes such substances.

(2) The acid is not irritative and has no sensitizing action, and therefore, a long-term continuous application and high-concentration application are possible.

(3) If the acid undergoes hydrolysis L-ascorbic acid is formed and the acid shows a reducing action and anti-oxidizing action, similar to those of ascorbic acid.

(4) The acid is easily hydrolyzed to L-ascorbic acid and D-glucose by an enzyme in the body, and physiological activities inherent to L-ascorbic acid are manifested.

(5) If L-ascorbic acid is orally taken together with an α-glycosyl saccharide compound or the like, the α-Glycosyl-L-ascorbic acid is formed in the body and metabolized. Accordingly, the safety factor is very high.

(6) In the case of a product containing a saccharide such as α-glucosyl saccharide compound , the effects of α-Glycosyl-L-ascorbic acid are exerted and the saccharide shows an excipient effect, a bulking effect and a sweetening effect. In the case of a purified product obtained by removing the saccharide, though the excipient effect and bulking effect are reduced, the inherent effects of α-Glycosyl-L-ascorbic acid are exerted with only a small amount.

Ethynylestradiol used in the present invention is a kind of a steroid hormone and is a compound in the form of a colorless needle crystal (recrystallized from dilute methanol) synthesized by ethynylation of estrone. The compound is insoluble in water but is soluble in ethanol, acetone, chloroform, dioxane and an ester.

The kojiic acid used in the present invention is 5-oxy-2-oxymethyl-γ-pyrrone produced from various carbohydrate compounds by Ascomycetes belonging to the genus Aspergillus or Penicillium. A product formed by synthesis also can be used. Refined kojiic acid is a colorless columnar crystal (recrystallized from acetone) having a molecular weight of 142.11, and is soluble in water, ethanol and an acetic acid ester but not easily soluble in ether, chloroform and pyridine.

As the placental extract used in the present invention, there can be mentioned dry powders, carbides, hydrolysis products and high-temperature extraction products of animal and human placental, and animal and human placental extract solutions. The process for preparing extract solutions is not particularly critical. For example, there can be mentioned an extract solution obtained by water-washing and finely pulverizing human placental, removing sufficient blood, adding refined water, and continuing the homogenization at a low temperature. As commercially available products, there can be mentioned a placentary kid (Kurt Richter supplied by Kotobuki Chemical) as an animal placental extract solution, Placenand V (supplied by Ichimaru Boeki) as a human placental extract solution, and Placene AF (supplied by Sansei Seiyaku).

The external dermatological composition of the present invention comprising α-Glycosyl-L-ascorbic acid having no direct reducing property provides excellent beautifying and whitening effects. Furthermore, the external dermatological composition comprising α-Glycosyl-L-ascorbic acid having no direct reducing property and ethynylestradiol, kojiic acid or placental extract in combination provides synergistically enhanced beautifying and whitening effects.

In the present invention, the amount of the α-Glycosyl-L-ascorbic acid incorporated as the solid is preferably 0.0005 to 10.0% by weight, most preferably 0.001 to 5.0% by weight, based on the total external dermatological composition.

In the present invention, the amount incorporated of kojiic acid or placental extract is preferably 0.0005 to 20.0% by weight, most preferably 0.001 to 10.0% by weight, based on the entire external dermatological composition.

In the present invention, ethynylestradiol is preferably incorporated in an amount of 0.00001 to 0.002% by weight based on the entire external dermatological composition.

As the amino acid used in the present invention, there can be mentioned neutral amino acids such as glycine, serine, cystine, alanine, threonine, cysteine, valine, phenylalanine, methionine, leucine, tyrosine, proline, isoleucine, tryptophan and hydroxyproline, acidic amino acids such as aspartic acid, asparagine, glutamine and glutamic acid, and basic amino acids such as arginine, histidine and lysine.

As the amino acid derivative, there can be mentioned not only acylsarcosine, salts thereof, acylglutamic acid, salts thereof, acyl-β-alanine, salts thereof, glutathione, pyrrolidone-carboxylic acid and salts thereof, but also oligopeptides such as glutazine, carnosine, glamcigine S, tyrocidine A and thyrocidine B.

The dead nettle extract used in the present invention is obtained by pulverizing stalks, leaves and flowers of dead nettles in a mixed extracting solution of water and a polar solvent such as 1,3-butylene glycol, propylene glycol or ethanol, extracting the pulverization product in the solution at room temperature, and concentrating the extraction product. This extract contains a polyphenol, a saccharide, an amino acid and the like, as effective ingredients. For example, as the commercially available product, there can be mentioned Extrait d'Ortie Blanche supplied by Somcichmie Co., France.

Glycyrrhizin is a compound having a structure similar to that of a steroid hormone, and is also called "glycyrrhetinic acid".

As the mucopolysaccharide used in the present invention, there can be mentioned mucopolysaccharides such as chondroitin-4-sulfate, chondroitin-6-sulfate, hyaluronic acid, dermatan sulfate, keratin sulfate, heparan sulfate and mucoithin acid, and salts thereof, and at least one thereof is appropriately selected and used.

In the present invention, preferably the α-Glycosyl-L-ascorbic acid having no direct reducing property is incorporated in an amount of 0.0005 to 10.0% by weight, especially 0.001 to 5.0% by weight, based on the entire external dermatological composition.

The allantoin which may be used in the present invention is a water-soluble and ether-insoluble nitrogen compound having a molecular weight of 158.

As the allantoin derivative which may be used in the present invention, there can be mentioned allantoic acid, dihydroxyaluminum allantonate, and chlorohydroxyaluminum allantoate.

The vitamin E which may be used in the present invention is an oily substance called "tocopherol".

As the vitamin E which may be derivative used in the present invention, there can be mentioned an acetic acid ester or vitamin E and a nicotinic acid ester of vitamin E. In general, the vitamin E derivatives are soluble in oils and fats, paraffin and organic solvents but not easily soluble or insoluble in water and aqueous organic solvents.

Also, in the present invention, preferably the amino acid or salt thereof, the dead nettle extract, glycyrrhizin, or the mucopolysaccharide and the additional possible allantoin or the derivative thereof, vitamine E or the derivative thereof, are incorporated in an amount of 0.0005 to 20.0% by weight, 0.001 to 10.0% by weight, based on the entire external dermatological composition.

In addition to the above-mentioned essential ingredients, the assistants described below can be incorporated, as long as they do not hinder an attainment of the intended effects.

As the anionic surface active agent, there can be mentioned anionic surface active agents having, in the molecule, at least one member selected from the group consisting of a carboxylic acid group, a sulfonic acid group, a sulfuric acid ester group and a phosphoric acid ester group. As the carboxylic acid group-containing surface active agent, there can be mentioned a fatty acid soap, an ether-carboxylic acid, a salt thereof, and a carboxylic acid salt of an amino acid-fatty acid condensate. As the surface active agent having a sulfonic acid salt, there can be mentioned an alkylsulfonic acid salt, sulfosuccinic acid, an estersulfonic acid salt, an alkylallyl-sulfonic acid salt, an alkylnaphthalene-sulfonic acid salt, an N-acylsulfonic acid and a formalin condensate-sulfonic acid salt. As the surface active agent having a sulfuric acid ester group, there can be used a sulfated oil, an ester-sulfuric acid salt, an alkylsulfuric acid salt, an ether-sulfuric acid salt, an alkylallylether-sulfuric acid salt and an amide-sulfuric acid. As the surface active agent having a phosphoric acid ester group, there can be mentioned an alkylphosphoric acid salt, an amide-phosphoric acid salt, an ether-phosphoric acid salt and an alkylallyletherphosphoric acid salt.

As the amphoteric surface active agent, there can be mentioned an alkylbetaine, an alkylamide-betaine, and an alkylimidazolinium-betaine.

As the semi-polar surface active agent, there can be mentioned dimethyllaurylamine oxide, dimethylmyristylamine oxide, dimethylacetylamine oxide, dimethylstearylamine oxide, dimethyloleylamine oxide, dimethylbehenylamine oxide, and methyldilaurylamine oxide. As the nonionic surface active agent, there can be mentioned a fatty acid alkanolamine, a polyoxyethylene fatty acid amide, and an alkanolamine. As the cationic surface active agent, there can be mentioned a fatty acid amine salt, an alkyl quaternary ammonium salt, an aromatic quaternary ammonium salt, a pyridium salt, and an imidazolium salt.

As the oleophilic nonionic surface active agent, there can be mentioned, for example, sorbitan fatty acid esters such as sorbitan monooleate, sorbitan monoisostearate, sorbitan monolaurate, sorbitan monopalmitate, sorbitan monostearate, sorbitan trioleate, diglycerolsorbitan penta-2-ethylhexylate and diglycerolsorbitan tetra-2-ethylhexylate, glycerol polyglycerol-fatty acids such as glycerol mono-cotton seed oil fatty acid, glycerol monoerucate, glycerol sesquioleate, glycerol monostearate, glycerol α'-oleate pyrogluatamate and glycerol monostearate malate, propylene glycol fatty acid esters such as propylene glycol monostearate, and a hardened castor oil derivative and glycerolalkyl ether.

As the hydrophilic nonionic surface active agent, there can be mentioned, for example, POE sorbitan fatty acid esters such as POE sorbitan monooleate, POE sorbitan monostearate, POE sorbitan monooleate and POE sorbitan tetraoleate, POE sorbitol fatty acid esters such as POE sorbitol monolaurate, POE sorbitol monooleate, POE sorbitol pentaoleate and POE sorbitol monostearate, POE glycerol fatty acid esters such as POE glycerol monostearate, POE glycerol monoisostearate and POE glycerol triisostearate, POE fatty acid esters such as POE monooleate, POE distearate, POE monodioleate and ethylene glycol distearate, POE alkylethers such as POE laurylether, POE oleylether, POE stearylether, POE behenylether, POE 2-octyldodecylether and POE cholestanolether, POE alkylphenylethers such as POE octylphenylether, POE nonylphenylether and POE dinonylphenylether, Pluronic type surface active agents such as Pluronic, POE/POP alkyl ethers such as POE/POP cetylether, POE/POP 2-decyltetradecylether, POE/POP monobutylether, POE/POP hydrous lanolin and POE/POP glycerolether, tetraPOE/tetraPOP-ethylenediamine condensates such as Tetronic, POE castor oil and POE hardened castor oil derivatives such as POE castor oil, POE hardened oil, POE hardened oil monoisostearate, POE hardened castor oil triisostearate, POE hardened castor oil monopyroglutamic acid-monoisostearic acid diester, POE bees wax-lanolin derivatives such as POE sorbitol bees wax, alkanolamides such as coconut oil fatty acid diethanolamide, lauric monoethanolamide and fatty acid isopropanolamide, and POE propylene glycol fatty acid esters, POE alkylamines, POE fatty acid amides, sucrose fatty acid esters, POE-nonylphenylformaldehyde condensates, alkylethoxydimethylamine oxides and trioleylphosphoric acid.

As the amino acid, there can be mentioned, for example, neutral amino acids such as glycine, alanine, valine, leucine, isoleucine, serine, threonine, phenylalanine, tyrosine, tryptophan, cystine, cysteine, methionine, proline, hydroxyproline and L-Dopa, acidic amino acids such as aspartic acid, glutamic acid, asparagine and glutamine, and basic amino acids such as arginine, histidine and lysine. As the amino acid derivative, there can be mentioned sodium acylsarcosine (sodium lauroylsarcosine), acylglutamic acid salts, sodium acyl-β-alanine, glutathione, and pyrrolidonecarboxylic acid.

As the silicone, there can be mentioned, for example, linear polysiloxanes such as dimethylpolysiloxane, methylphenylpolysiloxane and methylhydrodienepolysiloxane, cyclic polysiloxanes such as decamethylpolysiloxane, dodecamethylpolysiloxane and tetramethyltetrahydrodienepolysiloxane, and silicone resins capable of forming a three-dimensional crosslinked structure and silicone rubbers.

As the pearlescent agent, there can be mentioned, for example, natural fish scales, mica-titanium oxide composites, and bismuth oxychloride. Powders obtained by subjecting these substances to a hydrophilic or oleophilic treatment also can be used as the pearlescent agent.

As the higher alcohol, there can be mentioned, for example, linear alcohols such as lauryl alcohol, cetyl alcohol, stearyl alcohol, behenyl alcohol, myristyl alcohol, oleyl alcohol and cetostearyl alcohol, and branched alcohols such as monostearyglycerol ether (batyl alcohol), 2-decyltetradecinol, lanoline alcohol, choresterol, phytosterol, hexyl dodecanol, isostearyl alcohol, and octyl dodecanol.

As the higher fatty acid, there can be mentioned lauric acid, myristic acid, stearic acid, behenic acid (behenilic acid), oleic acid, 12-hydroxystearic acid, undecylenic acid, tallic acid, lanolin fatty acid, isostearic acid, linolic acid, linoleic acid, and eicosapentaenoic acid.

As the metal ion-sequestering agent, there can be mentioned, for example, 1-hydroxyethane-1,1-diphosphoric acid, tetrasodium 1-hydroxyethane-1,1-diphosphonate, disodium ethylenediamine tetraacetate tetrasodium ethylenediamine tetraacetate, sodium citrate, sodium polyphosphate, sodium metaphosphate and gluconic acid.

As the water-soluble polymer, there can be mentioned plant polymers such as gum arabic, tragacanth gum, galactane, gua gum, carob gum, karaya gum, carrageenan, pectin, agar, quinee seed (quinee), algae colloid (brown algae extract), starch (rice, corn, potato, wheat or the like) and glycyrrhetinic acid, microorganism polymers such as xanthane gum, dextran, succinoglucane and pullulan, and animal polymers such as collagen, casein, albumin and gelatin.

As the semi-synthetic water-soluble polymer, there can be mentioned, for example, starch polymers such as carboxymethylstarch and methylhydroxypropylstarch, cellulose polymers such as methylcellulose, nitrocellulose, ethylcellulose, methylhydroxypropylcellulose, hydroxyethylcellulose, sodium cellulose-sulfate, hydroxypropylcellulose, sodium carboxymethylcellulose (CMC), crystalline cellulose and cellulose powder, and alginic acid polymers such as sodium alginate and propylene glycol alginate.

As the synthetic water-soluble polymer, there can be mentioned, for example, vinyl polymers such as polyvinyl alcohol, polyvinylmethyl ether, polyvinylpyrrolidone and carboxyvinyl polymer (carbopol), polyoxyethylene polymers such as polyethylene glycol 20,000, polyethylene glycol 4,000,000 and polyethylene glycol 600,000, polyoxyethylenepolyoxypropylene copolymers, acrylic polymers such as sodium polyacrylate, polyethylene acrylate and polyacrylamide, and polyethylene-imine and cationic polymers.

As the inorganic water-soluble polymer, there can be mentioned, for example, bentonite, AℓMg silicate (bee gum), laponite, hectorite and silica.

As the synthetic ester, there can be mentioned, for example, isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanonyl acetate, isocetylstearate, isocetyl isostearate, cholesteryl 12-hydroxystearate, ethylene glycol di-2-ethylhexylate, dipentaerythritol fatty acid ester, N-alkylglycol monoisostearate, neopentyl glycol dicaprate, diisostearyl malate, glycerol di-2-heptylundecanoic acid, trimethylolpropane triisostearate, pentaerythritol tetra-2-ethylhexylatem, glycerol tri-2-ethylhexylate, trimethylolpropane triisostearate, cetyl 2-ethylhexanoate, 2-ethylhexyl palmitate, glycerol trimyristate, glyceride tri-2-heptylundecanoate, castor oil fatty acid methyl ester, oleyl oleate, cetostearyl alcohol, acetoglyceride, 2-heptylundecyl palmitate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, and triethyl citrate.

As the synthetic resin emulsion, there can be mentioned, for example, an acrylic resin emulsion, a polyethyl acrylate emulsion, an acryl resin solution, a polyacrylalkyl ester emulsion, and a polyvinyl acetate resin emulsion.

As the lower alcohol, there can be mentioned ethanol and isopropanol.

As the polyhydric alcohol, there can be mentioned, for example, dihydric alcohols such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol and octylene glycol, trihydric alcohols such as glycerol, trimethylolpropane and 1,2,6-hexane-triol, tetrahydric alcohols such as pentaerythritol, pentahydric alcohols such as xylitol, hexahydric alcohols such as sorbitol and mannitol, polyhydric alcohol polymers such as diethylene glycol, dipropylene glycol, triethylene glycol, polypropylene glycol, tetraethylene glycol, diglycerol, polyethylene glycol, triglycerol, tetraglycerol and polyglycerol, dihydric alcohol alkyl ethers such as ethylene glycol monomethyl ether, ethylene glycol monoethyl ether, ethylene glycol monobutyl ether, ethylene glycol monophenyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether and ethylene glycol dibutyl ether, dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether and dipropylene glycol butyl ether, dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol adipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate and propylene glycol monophenyl ether acetate, glycerol monoalkyl ethers such as xyl alcohol, selachyl alcohol and batyl alcohol, saccharide alcohols such as sorbitol, maltitol, maltotriose, mannitol, sucrose, erythritol, glucose, fructose, starch-decomposed saccharide, maltose, xylitose and starch-decomposed saccharide-reduced alcohol, and glycollide, tetrahydrofuryl alcohol, POE tetrahydrofuryl alcohol, POP butyl ether, POP/POE

6

EP 0 487 404 B1

butyl ether, tripolyoxypropylene glycerol ether, POP glycerol ether, POP glycerol ether phosphate, and POP/POE pentaerythritol ether.

As the thickening agents, there can be mentioned, for example, gum arabic, carrageenan, karaya gum, tragacanth gum, carob gum, quinee seed (quinee), casein, dextrin, gelatin, sodium pectinate, sodium arachate, methylcellulose, ethylcellulose, CMC, hydroxyethylcellulose, PVA, PVM, PVP, sodium polyacrylate, carboxyvinyl polymer, locust bean gum, gua gum, tamarind gum, cellulose dialkyldimethylammonium sulfate, xanthane gum, AℓMg silicate, bentonite, and hectorite.

As the oil component, there can be mentioned, for example, liquid oils and fats such as avocado oil, camellia oil, turtle oil, macademia nut oil, corn oil, mink oil, olive oil, rapeseed oil, yolk oil, sesame oil, parsic oil, wheat germ oil, sasanqua oil, castor oil, linseed oil, safflower oil, cottonseed oil, perilla oil soybean oil, peanut oil, teaseed oil, kaya oil, rice bran oil, Chinese tung oil, Japanese tung oil, jojoba oil, germ oil, triglycerol, glycerol trioctanoate and glycerol triisopalmitate, solid oils and fats such as cacao fat, coconut fat, horse tallow, hardened coconut oil, palm oil, beef tallow, mutton tallow, hardened beef tallow, palm nut oil, lard oil, beef bone tallow, sumac nut oil, hardened oil, beef leg tallow, Japan tallow and hardened castor oil, waxes such as bees wax, candelilla wax, cotton wax, carnauba wax, sperm wax, montan wax, bran wax, lanolin, kapok wax, lanolin acetate, liquid lanolin, sugar cane wax, isopropyl lanolin fatty acid, hexyl laurate, reduced lanolin, jojoba wax, hard lanolin, shellac wax, POE lanolin alcohol ether, POE lanolin alcohol acetate, POE chollesterol ether, polyethylene glycol lanolin fatty acid and POE hydrogenated lanolin alcohol ether, and hydrocarbons such as liquid paraffin, ozocerite, squalene, pristane, paraffin, ceresin, squalene, vaseline and micro-crystalline wax.

As the organic amine, there can be mentioned, for example, monoethanolamine, diethanolamine, triethanolamine, morpholine, triisopropanolamine, 1-amino-2-methyl-1,3-propane-diol and 2-amino-2-methyl-1-propanol.

As the inorganic powder, there can be mentioned, for example, talc, titanium oxide, kaolin, silica, silicate, zinc oxide, calcium carbonate, magnesium carbonate, Indian red, yellow iron oxide, chromium oxide, carbon black, ultramarine blue, mica, cerussite, nylon powder, polyethylene powder, cellulose powder, acrylic resin, titanium dioxide, and iron oxide.

As the inorganic pigment, there can be mentioned, for example, talc, kaolin, calcium carbonate, zinc powder, titanium dioxide, red iron oxide, yellow iron oxide, black iron oxide, ultramarine blue, titanium-coated mica, bismuth oxychloride, Indian red, binding pigment, ultramarine pink, chromium hydroxide, cobalt aluminum oxide, iron blue, black iron oxide, carbon black, silica, magnesium silicate, bentonite, mica zirconium oxide, magnesium oxide, zinc oxide, titanium oxide, light calcium carbonate, heavy calcium carbonate, light magnesium carbonate, heavy magnesium carbonate, and calamine.

As the polysaccharide, there can be mentioned, for example, cellulose, quinee seed, starch, galactan, glycogen, gum arabic, tragacanth gum, chondroitin, xanthane gum, gua gum, dextran, keratosulfate, locust bean gum, succinoglucane, and caronic acid.

As the ultraviolet absorbant, there can be mentioned, for example, benzoic acid type ultraviolet absorbants such as para-aminobenzoic acid (hereinafter referred to as "PAPB"), PABA monoglycerol ester, N,N-propoxy-PABA ethyl ester, N,N-diethoxy-PAPA ethyl ester, N,N-dimethyl-PABA methyl ester, N,N-dimethyl-PABA ethyl ester and N,N-dimethyl-PABA butyl ester, anthranilic acid type ultraviolet absorbants such as homomenthyl-N-acetyl anthranilate, salicylic acid type ultraviolet absorbants such as amyl salicylate, homomenthyl salicylate, octyl salicylate, phenyl salicylate, benzyl salicylate and p-isopropanolphenyl salicylate, cinnamic acid type ultraviolet absorbants such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-$\alpha$-cyano-$\beta$-phenyl cinnamate, 2-ethylhexyl-$\alpha$-cyano-$\beta$-phenyl cinnamate and glycerol mono-2-ethylhexanoyl-diparamethoxy cinnamate, benzophenone type ultraviolet absorbants such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone and 4-hydroxy-3-carboxybenzophenone, and 3-(4'-methylbenzylidene)-d,ℓ-camphor, 3-benzylidene)-d,ℓ-camphor, urocanic acid, ethyl urocanate, 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methylphenyl)benzotriazole, dibenzazine, dianisoylmethane, 4-methoxy-4'-t-butylbenzoylmethane and 5-(3,3-dimethyl-2-norbornylidene)-3-pentan-2-one.

As the clay mineral, there can be mentioned, for example, natural and synthetic water-swellable clay minerals such as montmorillonite, sakonite, nontronite, saponite, hectorite, vermiculite, bee gum, bentonite,

7

silicate, fluorosilicate, magnesium, aluminum and synthetic hectrite (Laponite).

Furthermore, the external dermatological composition of the present invention may comprise a perfume, an antiseptic agent, a fungicide, water, an antioxidant and the like, in addition to the foregoing ingredients.

Examples

The present invention will now be described in detail with reference to the following examples, that by no means limit the scope of the invention. In the examples, all of the incorporated amounts are by weight.

The beautifying and whitening effects were evaluated based on a test of a cumulative coating on the skin and a test of an elimination of blots and freckles.

Test of Beautifying and Whitening Effects by Cumulative Coatings

(Test Method)

A sample lotion was coated on the face every morning and every evening continuously for 2 months, by subjects troubled with dark skin, blots and freckles (each group consisting of 10 subjects), and after the two months' test, the beautifying and whitening effects were examined.

(Judgment Standard)

Conspicuously effective:
No substantial depositing of the pigment observed.
Effective:
Depositing of the pigment was moderated.
Slightly effective:
Depositing of pigment was slightly moderated.
Ineffective:
No change observed.
The results of the judgement are shown in Table 1 below.

Table 1

| + + : | The ratio (effect ratio) of the subjects for whom the sample was conspicuously effective or effective was at least 80%. |
|---|---|
| + : | The ratio (effect ratio) of the subjects for whom the sample was conspicuously effective or effective was at least 60% but lower than 80%. |
| ± : | The ratio (effect ratio) of the subjects for whom the sample was conspicuously effective or effective was at least 40% but lower than 60%. |
| - : | The ratio (effect ratio) of the subjects for whom the sample was conspicuously effective or effective was lower than 40%. |

Example 1 and Comparative Example 1 (Creams)

Creams were prepared by customary procedures according to the following recipes.

|  | Example 1 | Comparative Example 1 |
|---|---|---|
| Bees wax | 10.0 | 10.0 |
| Paraffin wax | 6.0 | 6.0 |
| Lanolin | 3.0 | 3.0 |
| Isopropyl myristate | 6.0 | 6.0 |
| Squalene | 8.0 | 8.0 |
| Liquid paraffin | 25.0 | 25.0 |
| 2-O-$\alpha$-D-glucosyl-L-ascorbic acid | 1.0 | - |
| Kojic acid | 0.005 | 0.005 |
| Polyoxyethylene sorbitan monostearate | 4.2 | 4.2 |
| Borax | 0.7 | 0.7 |
| Antiseptic agent | q.s. | q.s. |
| perfume | q.s. | q.s. |
| Distilled water | balance | balance |

The test of the beautifying and whitening effects by the cumulative coating was carried out for the creams obtained in Example 1 and Comparative Example 1. The results are shown in Table 1.

Table 1

|  | Example 1 | Comparative Example 1 |
|---|---|---|
| Beautifying and Whitening Effects | + + | ± |

From the results shown in Table 1, it is seen that the cream of Example 1 is an external dermatological composition having excellent beautifying and whitening effects.

Example 2 and Comparative Example 2 (Cosmetic Lotion

Cosmetic lotions were prepared by customary procedures according to the following recipes.

|  | Example 2 | Comparative Example 2 |
|---|---|---|
| Sorbitol (70%) | 3.0 | 3.0 |
| Glycerol | 5.0 | 5.0 |
| 2-O-$\alpha$-D-glucosyl-L-ascorbic acid | 0.5 | - |
| Ethynylestradiol | 0.00005 | 0.00005 |
| Sodium laurylsulfate | 0.01 | 0.01 |
| Ethyl alcohol | 20.0 | 20.0 |
| Antiseptic agent | q.s. | q.s. |
| Perfume | q.s. | q.s. |
| Distilled water | Balance | Balance |

The test of the beautifying and whitening effects by the cumulative coating was carried out for the cosmetic lotions obtained in Example 2 and Comparative Example 2. The results are shown in Table 2.

Table 2

| | Example 2 | Comparative Example 2 |
|---|---|---|
| Beautifying and Whitening Effects | + + | - |

It is seen that the cosmetic lotion obtained in Example 2 is an external dermatological composition having excellent beautifying and whitening effects.

Example 3 (Cosmetic Lotion)

A cosmetic lotion was prepared by customary procedures according to the following recipe.

| | |
|---|---|
| Sorbitol (70%) | 3.0 |
| Placentary kid | 0.5 |
| $\alpha$-Glycosyl-L-ascorbic acid having no direct reducing property[*1] | 0.5 |
| Dimethylstearylamine oxide | 0.05 |
| Sodium lauryl sulfate | 0.01 |
| Ethyl alcohol | 20.0 |
| Antiseptic agent | q.s. |
| Perfume | q.s. |
| Distilled water | balance |

Note

[*1]: $\alpha$-Glycosyl-L-ascorbic acid obtained in Example A-5 of Japanese Patent Application No. 01-27418

The cosmetic lotion of Example 3 had excellent beautifying and whitening effects, stability and safety, and was not sticky.

Example 4 (Cosmetic Lotion)

A cosmetic lotion was prepared by customary procedures according to the following recipe.

| | |
|---|---|
| Sorbitol (70%) | 3.0 |
| Glycerol | 5.0 |
| Ethynyl estradiol | 0.03 |
| $\alpha$-Glycosyl-L-ascorbic acid having no direct reducing property[*2] | 2.0 |
| POE (80 moles) hardened castor oil derivative | 0.5 |
| 4-Methoxy-4'-t-butyl dibenzoylmethane | 3.0 |
| Ethyl alcohol | 20.0 |
| Antiseptic agent | q.s. |
| Perfume | q.s. |
| Distilled water | balance |

Note

[*2]: $\alpha$-Glycosyl-L-ascorbic acid obtained in Example A-1 of Japanese Patent Application No. 01-27418

The cosmetic lotion of Example 4 had excellent beautifying and whitening effects and safety.

Example 5 (Cream)

A cream was prepared by customary procedures according to the following recipe.

| | |
|---|---|
| Bees wax | 10.0 |
| Paraffin wax | 6.0 |
| Lanolin | 3.0 |
| Isopropyl myristate | 6.0 |
| Squalene | 8.0 |
| Liquid paraffin | 25.0 |
| Kojiic acid | 0.5 |
| α-Glycosyl-L-ascorbic acid having no direct reducing property[*2] | 0.5 |
| N,N-Dimethyl-PABA-octyl ester | 5.0 |
| Polyoxyethylene sorbitan monostearate | 4.2 |
| Propylene glycol | 2.0 |
| Borax | 0.7 |
| Antiseptic agent | q.s. |
| Perfume | q.s. |
| Distilled water | balance |

Note
*2: α-Glycosyl-L-ascorbic acid obtained in Example A-1 of Japanese Patent Application No. 01-27418

The cream of Example 5 had excellent beautifying and whitening effects, stability, and safety.

Examples 6 through 9 and Comparative Example 3 (Creams)

The phase B shown in Table 3 was heated and maintained at 70°C, and the phase A was then added to this phase B to effect a preliminary emulsification. The mixture was then uniformly emulsified by a homogenizer, and the emulsion was gradually cooled to prepare a cream.

Table 3

| | Components | Example No. | | | | Comparative Example No. |
|---|---|---|---|---|---|---|
| | | 6 | 7 | 8 | 9 | 3 |
| Phase A | stearyl alcohol | 7.0 | 7.0 | 7.0 | 7.0 | 7.0 |
| | stearic acid | 2.0 | 2.0 | 2.0 | 2.0 | 2.0 |
| | chorosterol stearate | 2.0 | 2.0 | 2.0 | 2.0 | 2.0 |
| | squalene | 5.0 | 5.0 | 5.0 | 5.0 | 5.0 |
| | 2-octyldodecyl alcohol | 6.0 | 6.0 | 6.0 | 6.0 | 6.0 |
| | polyoxyethylene (25 moles) cetyl alcohol ether | 3.0 | 3.0 | 3.0 | 3.0 | 3.0 |
| | glyceryl monostearate | 2.0 | 2.0 | 2.0 | 2.0 | 2.0 |
| | vitamin E acetate | - | - | - | - | - |
| Phase B | propylene glycol | 5.0 | 5.0 | 5.0 | 5.0 | 5.0 |
| | hyaluronic acid | - | 1.0 | - | - | - |
| | glutamic acid | - | - | 0.5 | - | - |
| | dead nettle extract | - | - | - | 2.0 | - |
| | glycyrrhizin | 0.1 | - | - | - | - |
| | 2-O-α-D glucopyranosyl-L-ascorbic acid | 0.0005 | 0.0005 | 0.0005 | 0.0005 | - |
| | distilled water | balance | balance | balance | balance | balance |

One of the creams shown in Table 3 (Examples 6 through 9) was coated on right halves of faces of three subjects (women 20 to 50 years old), and the cream of Comparative Example 3 was coated on left halves of the faces of the subjects. This coating treatment was conducted twice a day and continued for 2 months. After the coating test, both the left and right sides of the faces were measured by the replica method or by using a skin impedance unit to determine the moistness and texture fineness of the skin.

The skin impedance unit comprises a main body including a device for measuring the resistance and capacitance by a high frequency wave, created by Masuda et al., and portions for detecting the resistance and capacitance, a cord having a length of 1 cm, and a cylindrical electrode attached to the top end of the cord.

The electrode comprises a central electrode having a diameter of 1 mm and a concentric peripheral electrode spaced by 1.5 mm from the central electrode and having an inner diameter of 4 mm. If the electrode is applied to the skin, a high frequency flows therethrough. Note, as the intensity of the high frequency is only several $\mu$A, the subject feels no irritation.

When the electrode is lightly touched to a part to be tested, the resistance abruptly rises to a certain value within 1 second. The reciprocal number of this resistance is called "conductance" and is expressed by the unit $\mu\Omega$. The conductance has a substantially proportional relationship to the water content in the surface of the skin. If the water content in the skin is high, it is judged that the moistness of the skin is good, and accordingly, the moistness of the skin can be evaluated based on an increase of the conductance.

The cream of Example 6 was compared with the cream of Comparative Example 3 with respect to the conductance at the part tested by the skin impedance test. The results are shown in Table 4.

Table 4

| Cream used at right side of face | Example 6 | | |
|---|---|---|---|
| Subject No. | 1 | 2 | 3 |
| Conductance at right side of face | 121 | 134 | 130 |
| Conductance at left side of face | 16 | 11 | 32 |

In Example 6, where the cream comprising glycyrrhezin and 2-O-$\alpha$-D-glucosyl-L-ascorbic acid was coated, the conductance value was significantly higher than the conductance value of the surface of the tested part of the left half of the face. This means that the cream of Example 6 provide a greater moistness of the skin.

The conductance was measured with respect to all of the subjects, in the same manner as described above. The conductance on the right side of the face was compared with the conductance of the left side of the face coated with the control cream (Comparative Example 3). When the conductance on the right side was higher by at least 70% than that on the left side, it was determined that the sample was conspicuously effective. When the conductance on the right side was higher by 50 to 70% than that on the left side, it was determined that the cream was slightly effective. When the increase of the conductance was smaller than 50%, it was determined that the sample was ineffective. The results are shown in Table 5 .

Separately, the fineness of the texture of the skin was measured by to the replica method, whereby a silicone rubber was applied closely to the skin to obtain a surface image, an epoxy resin was cast in the silicone rubber to obtain a reverse image, a surface roughness tester was scanned on the surface of the reverse image, and the state of the skin was examined. When undulations of the skin surface were large, it was judged that the texture was fine.

Replica images of all of the subjects were obtained, and the right side of each replica image was compared with the left side thereof. When undulations on the right side of the face were apparently larger than undulations on the left side, it was judged that the sample was conspicuously effective; when undulations on the right side of the face were slightly larger than undulations on the left side, it was judged that the sample was slightly effective; and when there was no difference in the undulations of the right and left sides of the face, it was judged that the sample was ineffective. The results are shown in Table 5 .

Table 5

| Effect | Evaluation | Example No. | | | |
|---|---|---|---|---|---|
| | | 6 | 7 | 8 | 9 |
| Moistness of skin | conspicuously effective<br>slightly effective<br>ineffective | 1<br>2<br>0 | 2<br>1<br>0 | 1<br>2<br>0 | 2<br>1<br>0 |
| Texture of skin | conspicuously effective<br>slightly effective<br>ineffective | 2<br>1<br>0 | 3<br>0<br>0 | 3<br>0<br>0 | 2<br>1<br>0 |

Creams were similarly prepared, according to recipes shown in Table 6 , and the moistness and the texture of the skin were measured. The results are shown in Table 7 .

14

Table 6

| | Components | Comparative Example No. | | | |
|---|---|---|---|---|---|
| | | 4 | 5 | 6 | 7 |
| Phase A | stearyl alcohol | 7.0 | 7.0 | 7.0 | 7.0 |
| | stearic acid | 2.0 | 2.0 | 2.0 | 2.0 |
| | choresterol stearate | 2.0 | 2.0 | 2.0 | 2.0 |
| | squalene | 5.0 | 5.0 | 5.0 | 5.0 |
| | 2-octyldodecyl alcohol | 6.0 | 6.0 | 6.0 | 6.0 |
| | polyoxyethylene (25 moles) cetyl alcohol ether | 3.0 | 3.0 | 3.0 | 3.0 |
| | glyceryl monostearate | 2.0 | 2.0 | 2.0 | 2.0 |
| | vitamin E acetate | | | | |
| Phase B | propylene glycol | 5.0 | 5.0 | 5.0 | 5.0 |
| | hyaluronic acid | - | 1.0 | - | - |
| | glutamic acid | - | - | 0.5 | - |
| | glycyrrhizin | 0.1 | - | - | - |
| | dead nettle extract | - | - | - | 2.0 |
| | 2-0-α-D-glucosyl-L-ascorbic acid | - | - | - | - |
| | distilled water | balance | balance | balance | balance |

Table 7

| Effect | Evaluation | Comparative Example No. | | | |
|---|---|---|---|---|---|
| | | 4 | 5 | 6 | 7 |
| Moistness of skin | conspicuously effective<br>slightly effective<br>ineffective | 0<br>0<br>3 | 0<br>0<br>3 | 0<br>0<br>3 | 0<br>0<br>3 |
| Texture of skin | conspicuously effective<br>slightly effective<br>ineffective | 0<br>0<br>3 | 0<br>1<br>2 | 0<br>0<br>3 | 0<br>0<br>3 |

From the foregoing results, it is seen that the external dermatological composition of the present invention has an excellent skin irritation-improving effect.

Example 10 (Emulsion)

An emulsion was prepared by customary procedures according to the following recipe.

| | |
|---|---|
| POE(20)POP(2) cetyl alcohol ether | 1.0 |
| Silicone KF96 (20 cs) (Shinetsu Kagaku) | 2.0 |
| Liquid paraffin | 3.0 |
| Propylene glycol | 5.0 |
| Glycerol | 2.0 |
| Ethyl alcohol | 5.0 |
| Carboxyvinyl polymer | 0.3 |
| Hydroxypropylcellulose | 0.1 |
| 2-Aminomethylpropanol | 0.1 |
| Vitamin E acetate | 1.0 |
| Dead nettle extract | 0.05 |
| $\alpha$-Glycosyl-L-ascorbic acid having no direct reducing property[1] | 3.0 |
| Antiseptic agent | q.s. |
| Perfume | q.s. |
| Distilled water | balance |

Note

[1]: $\alpha$-Glycosyl-L-ascorbic acid obtained in Example A-1 of Japanese Patent Application No. 01-27418

16

Example 11 (Emulsion)

An emulsion was prepared by customary procedures according to the following recipe.

| | |
|---|---|
| POE(20)POP(2) cetyl alcohol ether | 1.0 |
| Silicone KF96 (20 cs) (Shinetsu Kagaku) | 2.0 |
| Liquid paraffin | 3.0 |
| Propylene glycol | 5.0 |
| Glycerol | 2.0 |
| Ethyl alcohol | 15.0 |
| Carboxyvinyl polymer | 0.3 |
| Hydroxypropylcellulose | 0.1 |
| 2-Aminomethylpropanol | 0.1 |
| Hyaluronic acid | 0.05 |
| 2-O-$\alpha$-D-Glucosyl-L-ascorbic acid | 2.0 |
| Antiseptic agent | q.s. |
| Perfume | q.s. |
| Distilled water | balance |

Example 12 (Emulsion)

An emulsion was prepared by customary procedures according to the following recipe.

| | |
|---|---|
| Stearic acid | 2.0 |
| Cetanol | 1.0 |
| Vaseline | 3.0 |
| Lanolyl alcohol | 2.0 |
| Liquid paraffin | 8.0 |
| Squalene | 3.0 |
| Glycyrrhizin | 0.5 |
| Allantoin | 0.5 |
| Hyaluronic acid | 1.0 |
| $\alpha$-Glycosyl-L-ascorbic acid having no direct reducing property[*2] | 0.1 |
| POE(10) monooleate | 2.5 |
| Triethanolamine | 1.0 |
| Propylene glycol | 5.0 |
| Antiseptic agent | q.s. |
| Perfume | q.s. |
| Distilled water | balance |

Note
*2: $\alpha$-Glucosyl-L-ascorbic acid obtained in Example 4-A of Japanese Patent Application No. 01-27418

Example 13 (Nutritive Cream)

A nutritive cream was prepared by customary procedures according to the following recipe.

| | |
|---|---|
| Stearic acid | 2.0 |
| Stearyl alcohol | 7.0 |
| Reduced lanolin | 2.0 |
| Squalene | 5.0 |
| Octyl dodecanol | 6.0 |
| POE(25) cetyl ether | 3.0 |
| Glycerol monostearate | 2.0 |
| Antiseptic agent | q.s. |
| Perfume | q.s. |
| Propylene glycol | 5.0 |
| Dead nettle extract | 0.001 |
| 2-O-$\alpha$-D-glucosyl-L-ascorbic acid | 1.0 |
| Distilled water | balance |

Example 14 (Emulsion)

An emulsion was prepared by customary procedures according to the following recipe.

| | |
|---|---|
| POE(20)POP(2) cetyl alcohol ether | 1.0 |
| Silicone KF96 (20 cs) (Shinetsu Kagaku) | 2.0 |
| Liquid paraffin | 3.0 |
| Propylene glycol | 5.0 |
| Glycerol | 2.0 |
| Ethyl alcohol | 5.0 |
| Carboxyvinyl polymer | 0.3 |
| Hydroxypropylcellulose | 0.1 |
| 2-Aminomethylpropanol | 0.1 |
| Chondroitin sulfate | 0.1 |
| 2-O-$\alpha$-D-glucosyl-L-ascorbic acid | 1.0 |
| Antiseptic agent | q.s. |
| Perfume | q.s. |
| Distilled water | balance |

Example 15 (Astringent Cosmetic Lotion)

An astringent cosmetic lotion was prepared by customary procedures according to the following recipe.

| | |
|---|---|
| Dipropylene glycol | 2.0 |
| Citric acid | 0.03 |
| Sodium citrate | 0.05 |
| $\alpha$-Glycosyl-L-ascorbic acid having no direct reducing property[*3] | 0.001 |
| Histidine | 5.0 |
| Ethyl alcohol | 15.0 |
| Polyoxyethylene (15 moles added) oleyl alcohol ether | 0.5 |
| Antiseptic agent | q.s. |
| Perfume | q.s. |
| Distilled water | balance |

Note
[*3]: $\alpha$-Glucosyl-L-ascorbic acid obtained in Example A-5 of Japanese Patent Application No. 01-27418

Each of the external dermatological compositions of Examples 10 through 15 had an excellent skin irritation-improving effect.

As apparent from the foregoing description, in the external dermatological composition of the present invention, by incorporating $\alpha$-Glycosyl-L-ascorbic acid having no direct reducing property, with other medicinal components, the whitening and beautifying effects can be enhanced and a high skin irritation-improving effect can be attained.

**Claims**

1. An external dermatological composition having whitening effects comprising (i) $\alpha$-glycosyl-L-ascorbic acid having no direct reducing property and (ii) at least one member selected from the group consisting of ethinylestradiol, kojiic acid, placental extract, amino acids and derivatives thereof, dead nettle extract, glycyrrhizin, and mucopolysaccharides, wherein respective contents of the components (i) and (ii) exluding ethinylestradiol, are 0.0005 to 10.0% by weight and 0.0005 to 20.0% by weight, and the content of ethinylestradiol is 0.00001 to 0.03% by weight.

2. An external dermatological composition as claimed in claim 1, wherein the $\alpha$-glycosyl-L-ascorbic acid having no direct reducing property is 2-O-$\alpha$-D-glucosyl-L-ascorbic acid.

3. An external dermatological composition as claimed in claim 1, wherein the respective contents of the components (i) and (ii) excluding ethinylestradiol are 0.001 to 5.0% by weight and 0.001 to 10% by weight, and the content of ethinylestradiol is 0.00001 to 0.002% by weight.

4. An external dermatological composition as claimed in claim 1, further comprising (iii) at least one member selected from the group consisting of vitamin E and the derivatives thereof.

**Patentansprüche**

1. Äußerlich anzuwendende dermatologische Zusammensetzung mit Weißmachereffekten mit (i) $\alpha$-Glycosyl-L-ascorbinsäure, die keine direkte reduzierende Eigenschaft aufweist und (ii) mindestens einem Teil, ausgewählt aus der Gruppe bestehend aus Ethinylestradiol, Kojisäure, Plazentaextrakt, Aminosäuren und Derivaten hiervon, Taubnesselextrakt, Glycyrrhizin und Mucopolysacchariden, wobei die entsprechenden Gehalte der Komponenten (i) und (ii) ausschließlich Ethinylestradiol bei 0,0005 bis 10,0 Gew.-% bzw. 0,0005 bis 20,0 Gew.-% liegen, und der Gehalt an Ethinylestradiol bei 0,00001 bis 0,03 Gew.-% liegt.

2. Äußerlich anzuwendende dermatologische Zusammensetzung nach Anspruch 1, in der die $\alpha$-Glycosyl-L-ascorbinsäure mit keiner direkten reduzierenden Eigenschaft 2-O-$\alpha$-D-glucosyl-L-ascorbinsäure ist.

3. Äußerlich anzuwendende dermatologische Zusammensetzung nach Anspruch 1, in der die entsprechenden Gehalte der Komponenten (i) und (ii) ausschließlich Ethinylestradiol bei 0,001 bis 5,0 Gew.-% bzw. 0,001 bis 10,0 Gew.-% liegen, und der Gehalt an Ethinylestradiol bei 0,00001 bis 0,002 Gew.-% liegt.

4. Äußerlich anzuwendende dermatologische Zusammensetzung nach Anspruch 1, weiter enthaltend (iii) mindestens ein Teil, ausgewählt aus der Gruppe bestehend aus Vitamin E und den Derivaten hiervon.

**Revendications**

1. Composition dermatologique externe, ayant des effets d'éclaircissement, comprenant (i) de l'acide $\alpha$-glycosyl-L-ascorbique n'ayant aucune propriété réductrice directe, et (ii) au moins un membre du groupe consistant en éthynylestradiol, acide kojiique, extrait placentaire, aminoacides et leurs dérivés, extrait d'ortie blanche, glycyrrhizine et mucopolysaccharides, dans laquelle les quantités respectives des constituants (i) et (ii), à l'exclusion de l'éthynylestradiol, vont de 0,0005 à 10,0 % en poids et de 0,0005 à 20,0 % en poids, et la quantité d'éthynylestradiol va de 0,00001 à 0,03 % en poids.

2. Composition dermatologique externe suivant la revendication 1, dans laquelle l'acide $\alpha$-glycosyl-L-ascorbique n'ayant aucune propriété réductrice directe est l'acide 2-O-$\alpha$-D-glucosyl-L-ascorbique.

3. Composition dermatologique externe suivant la revendication 1, dans laquelle les quantités respectives des constituants (i) et (ii), à l'exclusion de l'éthynylestradiol, vont de 0,001 à 5,0 % en poids et de 0,001 à 10 % en poids, et la quantité d'éthynylestradiol va de 0,00001 à 0,002 % en poids.

4. Composition dermatologique externe suivant la revendication 1, comprenant en outre (iii) au moins un membre du groupe consistant en la vitamine E et ses dérivés.

20